# EUROPEAN PATENT APPLICATION

(11) **EP 2 581 363 A1**
(43) Date of publication of application: **17.04.2013**
(21) Application number: 11008323.5
(22) Date of filing: 14.10.2011
(51) Int. Cl.: C07C 51/58, C07C 53/48, C07C 53/38

(54) **Liquid phosgenation reagent comprising triphosgene and diphosgene**

(71) Applicant: Lonza Ltd, 3930 Visp (CH)
(72) Inventor: The designation of the inventor has not yet been filed

(57) **Abstract**

Preparation and use of a liquid phosgenation reagent.

## Description

The invention is directed to a liquid mixture comprising diphosgene and triphosgene and its use for example in phosgenation reactions.

The use of phosgene, diphosgene and triphosgene in preparative chemistry, in so called "phosgenation" reactions, is well known to the skilled person and is broadly described in the scientific literature, in many patent applications and even in text books. Phosgene and its higher homologues are often used for introduction of carbonyl groups (formation of carbamates from amines and alcohols), chlorination or dehydratisation. Phosgene and its higher homologues react with amines, alcohols and carboxylic acids. They are used for example for the preparation of urea, isocyanates, chloroformates, carbamates, polycarbonates and metal chlorides and intermediates for the preparation of dyes and herbicides.

Due to their state at standard conditions (1 bar, 20 °C) Phosgene (COCl₂, CAS [75-44-5], gaseous) and its higher homologues diphosgene (Cl-C(O)-OCCl₃, [503-38-8], liquid) and triphosgene (Cl₃CO-C(O)-OCCl₃, [32315-10-9], solid) are used as pure compounds. A higher homologue of phosgene than triphosgene is not commercially available.

We are not aware of a single example in the state of the art where the use of a mixture has been described. Triphosgene is known to be soluble in alcohol, benzene, ether, hexane, and tetrahydrofuran. The handling of all phosgene homologues is dangerous. Gaseous phosgene can be handled in pipes near its production facilities of as liquefied gas in tanks. It has a very high vapour pressure. Liquid diphosgene can be handled easily but has a high vapour pressure of 13,73 hPa at 20 °C. Triphosgene has a lower vapour pressure but causes dust problems during handling. Diphosgene has a density at standard conditions of 1.65 g/mL. The particle density of triphosgene is also 1.6 g/mL, whereas its bulk density is substantially lower. All phosgene derivatives are dangerous poisons because of formation of HCl in the lungs and tissue of all humans and animals.

Surprisingly we found that triphosgene has a remarkable solubility in diphosgene and the density of the resulting liquid solution is significantly higher than the density of both pure compounds, respectively. Also safety is increased for handling, since the inventive solution can be prepared directly in a plant while the solution can be shipped in tanks or bottles ready to use. The maximum density we reached in a solution of triphosgene in diphosgene at standard conditions was 1.85 g/mL.

The table below demonstrates below that the same amounts of pure diphosgene or triphosgene needs a 13% or at least 16% higher volume, respectively, compared to the same amount of the inventive solution having a concentration of 40.5 g triphosgene in 100 g diphosgene, which is near the upper limit of solubility.

The inventive solution can be obtained by mixing solid triphosgene to liquid diphosgene under stirring up to a concentration of 41 g triphosgene per 100 g diphosgene at room temperature. No cooling is necessary.

An advantage of the inventive solution compared to diphosgene is a reduced vapour pressure, which reduces the risk of hazards while handling the liquids. Furthermore, due to increased density of the inventive solution compared to pure diphosgene or triphosgene, the turnover of bottles or tanks can be reduced at the same activity.

Transportation of bulk amounts for example in wagons and tank lorries requires reduced amounts of vehicles, so even the danger of environmental hazards is reduced.

| | Mol weight | Density | Mass | Mol Amount | Phosgene equiv. | Action equiv. | Volume |
|---|---|---|---|---|---|---|---|
| Sample | [g/mol] | [g/mL] | [g] | [mmol] | [eq/mol] | [eq] | [mL] |
| Phosgene | 98.92 | gasous | | | 1 | | |
| diphosgene | 197.82 | 1.64 (liquid) | 5.6 | 28.309 | 2 | 56.617 | 3.414 |
| triphosgene | 296.74 | 1.6 (solid) | 2.27 | 7.650 | 3 | 22.949 | 1.419 |
| **40.5%-w/w mixture** | | **1.85** | **7.87** | | | **79.567** | **4.254** |
| diphosgene | 197.82 | 1.64 | 7.87 | 39.784 | 2 | 79.567 | **4.799** |
| triphosgene | 296.74 | 1.6 | 7.87 | 26.522 | 3 | 79.564 | **4.919** |

Thus we claim a liquid solution of triphosgene in diphosgene at a concentration of 0.1 to 41 g triphosgene per 100 g diphosgene, having a density in the range from 1.65 to 1.85 g/mL at standard conditions (1 bar, 20 °C).

In a preferred embodiment the concentration of triphosgene is from 1 to 41 g triphosgene per 100 g diphosgene, more preferably from 10 to 41 g triphosgene per 100 g diphosgene, even more preferred from 20 to 41 g triphosgene per 100 g diphosgene.

Use of the mixture above in preparative chemistry. In a preferred embodiment said mixture is used in phosgenation reactions.

The following examples demonstrate the equivalent use of a triphosgene/diphosgene solution compared to use of isolated diphosgene or triphosgene. The inventive solution has the advantage of reduced storage needs, and thus a higher action density the diphosgene or triphosgene.

### Examples

### Preparation of (S)-2-(2-Amino-5-chlorophenyl)-1,1,1-trifluorooct-3-yn-2-ol methanesulfonate (2:3 mol/mol)

A solution of (1*R*,2*S*)-PNE (17.6%-w/w, 42.0 g, 36.0 mmol) in THF/toluene (9:1-w/w) was charged a vessel at room temperature. A solution of diethylzinc in toluene (29.9%-w/w, 12.0 g, 29.05 mmol) was added at 17 to 25 °C and the mixture was aged at said temperature for 30 min, 1-hexyne (97%-w/w, 13.21 g, 156.0 mmol) was added at 18 °C and the resulting solution was aged at 20 °C for 60 min. A solution of BuLi in toluene (3.09 mol/kg, 35.53 g, 109.8 mmol) and a solution of 1-(2-amino-5-chlorophenyl)-2,2,2-trifluoroethanone (CN23315) in toluene/THF (1:1 w/w) (39.6%-w/w, 67.75 g, 120.0 mmol) were added in parallel to the reaction mixture at 20 °C within 3 h. The addition of BuLi was started 10 min in advance of the CN23315 addition. After completed addition of CN23315 the reaction mixture was stirred for 30 min at 20 °C, then heated to 30 °C over a period of 60 min and aged for 6 h at 30 °C. The reaction mixture was stirred at 0 °C overnight. HPLC indicated 81.9% conversion. The reaction mixture was diluted with toluene (51.6 g) and quenched by addition of aqueous citric acid solution (1 M, 88.2 g). After stirring for 15 min the phases were separated and the organic phase successively washed with water (18.1 g), aqueous NaHCO₃ solution (5%-w/w, 48.0 g) and water (24.0 g). The organic phase was partially concentrated (110 g residual solution), diluted with toluene (60 g), and partially concentrated again (114 g residue). The residue was diluted with toluene (120 g). Isopropyl alcohol (3.2 g) was added. Methanesulfonic acid (10.96 g, 114 mmol) was added at 30 °C over a period of 30 min and the mixture was stirred for 30 min. A second portion methanesulfonic acid (5.78 g, 60 mmol) was added at 30 °C over a period of 30 min. The mixture was stirred at 30 °C for 30 min, cooled to 5 °C over a period of 60 min, and aged at 5 °C for 30 min. The crystals were filtered, washed with cold toluene/isopropyl alcohol (98:1, 1×25 mL, 2×120 mL) and dried under vacuum at 40°C. The product (MSA salt of compound of formula II, wherein R¹ is trifluoromethyl, R² is hex-1-ynyl, R⁸ is 5-chloro, R⁹ is hydrogen and R¹⁰ is hydrogen, 28.57 g) was obtained as slightly beige solid (96.5%w-/w assay according to ¹H-NMR).

### Example 1: (S)-6-Chloro-4-(hex-1-ynyl)-4-(trifluoromethyl)-1,4-dihydro-2H-3,1-benzoxazin-2-one, reaction with triphosgene in diphosgene (40.5%-w/w)

(*S*)-2-(2-Amino-5-chlorophenyl)-1,1,1-trifluorooct-3-yn-2-ol methanesulfonate ((*S*)-CN47583) obtained according to example 20.2 (96.5%-w/w as methanesulfonate 2:3 mol/mol, 15.0 g, 32.2 mmol) in ethyl acetate/heptanes (2:1-w/w, 30 g) was charged to a jacketed 150 mL-reactor with agitator and off-gas scrubber with caustic soda. The reaction mixture was cooled to 15 °C and aqueous Na₂CO₃ solution (12%-w/w, 27 g, formation of gas during addition!) was added, and then the mixture was stirred for 5 min at 15 °C. The aqueous phase was removed. And aqueous Na₂CO₃ solution (12%-w/w, 33 g) was added to the organic phase. A solution of triphosgene (0.73 g, 2.5 mmol) in diphosgene (2.90 g, 14.7 mmol) was added to the reaction mixture over a period of 30 min at 7 to 11°C. The reaction mixture was stirred at 8 °C for 20 min. The reaction mixture was sampled for conversion control until a conversion of more than 99% was reached. The phases were allowed to separate. The aqueous phase was removed. The organic phase was dried over MgSO₄, filtered and concentrated to dryness. The crude product (10.5 g, 31.1 mmol, 97% yield) was obtained as yellow solid (purity >99.0% by HPLC, 98.6%-w/w assay by ¹H-NMR). The crude product was slurried in hexane (10 mL) for 3 h at room temperature. The product was filtered, washed with cold (approx. 5 °C) hexane (5 mL) and dried at 30 °C under vacuum. The product ((S)-compound of formula I, wherein R¹ is trifluoromethyl, R² is hex-1-ynyl, R⁸ is 6-chloro, R⁹ is hydrogen and R¹⁰ is hydrogen) was obtained as white solid (8.25 g, 24.6 mmol, 77% yield) with a purity of more than 99.0% and assay 99.1%-w/w according to ¹H-NMR. Concentration of the mother liquor to dryness under vacuum afforded additional product as yellow solid (1.58 g) with a purity of 97%.

### Preparation of (R)-2-(2-Amino-5-chlorophenyl)-1,1,1-trifluorooct-3-yn-2-ol methanesulfonate (2:3 mol/mol)

A solution of (1*S*,2*R*)-PNE (18.7%-w/w, 19.7 g, 18.0 mmol) in THF/toluene (9:1-w/w) was charged to a vessel at room temperature. A solution of diethylzinc in toluene (29.9%-w/w, 6.10 g, 14.8 mmol) was added at 17 to 25°C and the mixture was aged at said temperature for 30 min, 1-hexyne (97%-w/w, 6.10 g, 72.0 mmol) was added at 18°C and the resulting solution was aged at 20 °C for 60 min. A solution of BuLi in toluene (3.09 mol/kg, 17.8 g, 55.0 mmol) and a solution of 1-(2-amino-5-chlorophenyl)-2,2,2-trifluoroethanone (CN23315) in toluene/THF (1:1 w/w) (39.6%-w/w, 33.8 g, 60.0 mmol) were added in parallel to the reaction mixture at 20 °C within 3 h. The addition of BuLi was started 10 min in advance of the CN23315 addition. After completed addition of CN23315 the reaction mixture was stirred for 30 min at 20°C, then heated to 30 °C over a period of 60 min and aged for 6 h at 30°C. The reaction mixture was stirred at 0 °C overnight. HPLC indicated 89.6% conversion. The reaction mixture was diluted with toluene (25.8 g) and quenched by addition of aqueous citric acid solution (1 M, 44.1 g). After stirring for 15 min the phases were separated and the organic phase successively washed with water (9.1 g), aqueous NaHCO₃ solution (5%-w/w, 24.0 g) and water (12.0 g). The organic phase was partially concentrated (51 g residual solution), diluted with toluene (30 g), and partially concentrated again (58 g residue). The residue was diluted with toluene (59 g) and isopropyl alcohol (1.50 g). Methanesulfonic acid (10.48 g, 114 mmol) was added at 30 °C over a period of 30 min and the mixture was stirred for 30 min. A second portion methanesulfonic acid (2.89 g, 30 mmol) was added at 30 °C over a period of 30 min. The mixture was stirred at 30 °C for 30 min, cooled to 5 °C over a period of 60 min, and aged at 5 °C for 30 min. The crystals were filtered, washed with cold toluene (10 g) and dried under vacuum at 40 °C. The crude product (19.3 g) was obtained as yellowish solid with a purity of 93.3% and 99.6% ep. The product was further purified by slurring it in a mixture of toluene (100 mL) and isopropyl alcohol (2 mL) at room temperature for 3 h. The product (MSA salt of (R)-CN47583, compound of formula II, wherein R¹ is trifluoromethyl, R² is hex-1-ynyl, R⁸ is 5-chloro, R⁹ is hydrogen and R¹⁰ is hydrogen) was filtered, washed with toluene (10 mL) and dried at 40 °C under vacuum. The product was obtained as white solid (17.1 g, 35.3 mmol, 59% yield) with a purity of 93.3% and 99.9% ep, and an assay of 92.8%w-/w according to ¹H-NMR.

### Comparison Example 1: (R)-6-Chloro-4-(hex-1-ynyl)-4-(trifluoromethyl)-1,4-dihydro-2H-3,1-benzoxazin-2-one (Reaction with triphosgene)

(*R*)-2-(2-Amino-5-chlorophenyl)-1,1,1-trifluorooct-3-yn-2-ol methanesulfonate ((*R*)-CN47583) obtained according to example 20.1 (92.8%-w/w as methanesulfonate 2:3 mol/mol, 15.0 g, 30.9 mmol) in ethyl acetate/heptanes (2:1-w/w, 30 g) was charged to a jacketed 150 mL-reactor with agitator and off-gas scrubber with caustic soda. The reaction mixture was cooled to 15 °C and aqueous Na₂CO₃ solution (12%-w/w, 27 g, formation of gas during addition!) was added, and then the mixture was stirred for 5 min at 15 °C. The aqueous phase was separated and removed. Aqueous Na₂CO₃ solution (12%-w/w, 33 g) was added to the organic phase. Triphosgene (3.62 g, 12.2 mmol) was added in portions over a period of 25 min at 7 to 15 °C. The reaction mixture was stirred for 15 min at 8 °C and sampled for conversion control (conversion more than 99%). The phases were separated. The organic phase was dried over MgSO₄, filtered and concentrated under vacuum to dryness. The crude product (11.4 g) was obtained as yellow oil (purity more than 99.0%). A sample was cooled to 5 °C and it slowly solidified. The crude product was slurried in hexane (10 mL) for 2 h at room temperature. The product was filtered, washed with cold (approx. 5°C) hexane (5 mL) and dried at 30 °C under vacuum. The product ((R)-compound of formula I, wherein R¹ is trifluoromethyl, R² is hex-1-ynyl, R⁸ is 6-chloro, R⁹ is hydrogen and R¹⁰ is hydrogen) was obtained as white solid (7.73 g, 22.7 mmol, 73% yield) with a purity of more than 99.0% and an assay of 97.1 %-w/w according to ¹H-NMR. Concentration of the mother liquor to dryness under vacuum afforded additional product as yellow solid (2.54 g, 7.2 mmol, 23% yield) with a purity of 98% and an assay of 93.6%-w/w according to ¹H-NMR.

## Claims

1. A liquid solution of triphosgene in diphosgene at a concentration of 0.1 to 41 g triphosgene per 100 g diphosgene, having a density in the range from 1.65 to 1.85 g/mL at standard conditions (1 bar, 20 °C).

2. The solution of claim 1, having a concentration of I to 41 g triphosgene per 100 g diphosgene.

3. The solution of claims 1 or 2, having a concentration of 10 to 41 g triphosgene per 100 g diphosgene.

4. The solution of any of claims 1 to 3, having a concentration of 20 to 41 g triphosgene per 100 g diphosgene.

5. Use of a solution of any of claims 1 to 4 in preparative chemistry.

6. The use of claim 5 in phosgenation reactions.
